# EUROPÄISCHE PATENTSCHRIFT

(11) **EP 0 570 929 B1**
(45) Veröffentlichungstag und Bekanntmachung des Hinweises auf die Patenterteilung: **28.06.1995**
(21) Anmeldenummer: 93108127.7
(22) Anmeldetag: 18.05.1993
(51) Int. Cl.: A61B 17/60, A61F 2/44

(54) **Implantat für die Wirbeläule**
Implant for the spine
Implant pour le rachis

(30) Priorität: 18.05.1992 CH 1585/92
(43) Veröffentlichungstag der Anmeldung: 24.11.1993
(73) Patentinhaber: PINA VERTRIEBS AG, 8212 Neuhausen 2/SH (CH)
(72) Erfinder: Müller, Walter, D-7243 Vöhringen (DE); Piotrowski, Georg, D-7238 Oberndorf (DE)
(74) Vertreter: Neymeyer, Franz, Dipl.-Ing. (FH)

(56) Entgegenhaltungen:
- DE-C- 3 807 346
- DE-U- 8 711 992
- FR-A- 2 651 992
- US-A- 4 771 767
- US-A- 5 057 109

## Beschreibung

Die Erfindung betrifft ein mehrteiliges Implantat für die Wirbelsäule zur Fixierung der Wirbel, insbesondere der Halswirbel, auf der Vorderseite, d.h. speiseröhrenseitig, nach dem Oberbegriff des Anspruchs 1.

Es ist bereits ein mehrteiliges Implantat der gattungsgemäßen Art bekannt (US-4 771,767), das aus mehreren Montageblöcken besteht, die durch Gewindespindeln und Schwenklager miteinander verbunden sind. Dabei haben die Gewindespindeln jeweils ein lingsgängiges und ein rechtsgängiges Gewinde und dazwischen ein z.B. sechskantiges Schlüsselprofil zur Betätigung der Gewindespindel mittels eines Schraubenschlüssels od. dgl.
Diese bekannten Implantate bestehen, wie gesagt, aus Montageblöcken, die zweiteilig aufgebaut sind und nur eine einzige Bohrung zur Aufnahme einer einzigen Befestigungsschraube aufweisen, die in den Wirbelknochen eingeschraubt wird. Dabei bestehen die Blöcke aus jeweils zwei deckungsgleichen Blockhälften, die auf den einander zugekehrten Seiten Ausnehmungen zur schwenkbaren Aufnahme eines zylindrischen Lagerbolzens aufweisen, der mit einer quer verlaufenden Gewindebohrung oder einer kugelförmigen Gewindemutter versehen ist, in welche jeweils ein Teil einer Gewindespindel eingeschraubt werden kann. Zusammengehalten werden diese beiden Blockhälften jeweils durch einen die gemeinsame Bohrung durchragenden Gewindeschaft, der Bestandteil der Knochenschraube ist und auf den eine Gewindemutter spannend aufgeschraubt wird.
Weil nur eine einzige Knochenschraube für jeden Montageblock verwendet werden kann, besteht die Gefahr, daß sich die Montageblöcke bei der Betätigung der Gewindespindel zum Zwecke einer Kompression oder einer Distraktion um die Achse der Knochenschrauben verdrehen und somit eine exakte Fixierung in Frage stellen bzw. unmöglich machen.
Außerdem sind diese bekannten Implantate nur geeignet, dorsal, d.h. rückseitig, an den Wirbeln der Wirbelsäule befestigt zu werden, wobei es unumgänglich ist, zuvor die rückseitigen, fingerartigen Wirbelvorsprünge vom Wirbel zu entfernen, an dem ein Montageblock befestigt werden soll.
Für den erfindungsgemäßen Zweck, nämlich die cervikale, ventrale Anbringung an der Wirbelsäule sind diese bekannten Implantate jedenfalls nicht geeignet.

Ein anderes bekanntes Implantat besteht im wesentlichen aus einem Metallband, an welchem seitliche Laschen angeordnet sind, wobei sowohl das Metallband als auch die Laschen Schraubenbohrungen aufweisen, welche zur Aufnahme von Knochenschrauben dienen. Diese Metallbänder gibt es in verschiedenen Längen, damit sie entsprechend den körperlichen Verhältnissen bei einem Patienten angepaßt ausgewählt werden können.
Die Fixation der Wirbel erfolgt mit solchen bandartigen Implantaten durch Anschrauben des Implantates an einen Wirbel, nachdem an dessen Oberfläche ein sog. Bett für das Implantat dadurch geschaffen wurde, daß der Wirbelknochen W beispeilsweise abgefräst wird, um eine dem bandartigen Implantat entsprechende Auflagefläche herzustellen. Ebenso muß auch auf dem benachbarten, zu fixierenden Wirbel ein Bett geformt werden, um hernach diesen Wirbel mit dem bandartigen Implantat in der medizinisch erforderlichen Wirbelposition zu verschrauben.

Nachteilig bei einem solchen bandartigen Implantat ist, daß diese Vorgänge unter Operationsbedingungen auszuführen sind und daß auch noch die bei der Herstellung der beiden Betten für die bandartigen Implantate anfallenden Knochenspäne und dgl. beseitigt werden müssen. Darüberhinaus stehen die Köpfe der Knochenschrauben in der Regel mitsamt dem bandartigen Implantat vom Wirbel ab, was insbesondere im Bereich der Speiseröhre unangenehme, Beschwerden verursachende Begleiterscheinungen ergibt. Außerdem kann es je nach Lage der Wirbel oder deren Beschädigung erforderlich sein, zwischen dem bandartigen Implantat und dem einen oder beiden Wirbeln eine Unterlegscheibe als Distanzstück vorzusehen, um eine medizinisch geeignete Fixation zu erreichen. Insbesondere ist der Ort zum Eindrehen der Knochenschrauben nicht allein nach medizinischen Gesichtspunkten wählbar, weil die Schraubenlöcher im bandartigen Implantat fest vorgegeben sind, wodurch die Fixation nicht immer medizinisch optimal ausgeführt werden kann. Dies kann insbesondere im Fall einer Osteoporose zur Einschränkung der medizinisch erforderlichen Maßnahmen führen, da dann die Lage der Schraubenlöcher so gewählt werden muß, daß diese nicht ausbrechen. Insbesondere besteht die Gefahr des Ausbrechens auch bei Schraubenlöchern in Randzonen des Wirbels, welche oft bei dem bandartigen Implantat benutzt werden müssen, weil eben die Schraubenlöcher durch deren Abstände auf dem bandartigen Implantat fest vorgegeben sind. Darüberhinaus ist eine Lagerhaltung der verschiedenen, medizinisch erforderlichen Größen des bandartigen Implantats erforderlich.

Aufgabe der Erfindung ist die Behebung der Nachteile der bekannten Implantate und die Schaffung eines Implantates, welches bei minimaler oder völlig entfallender Vorbereitung bzw. Vorbearbeitung des Wirbelknochens die medizinisch konforme Fixation bzw. eine ventrale, cervikale Stabilisierung der Wirbel bei möglichst optimaler Wahl der Lage der Knochenschrauben auch unter Vermeidung von Behinderungen des Patienten, beispielsweise durch Beeinträchtigung des Speiseröhrenquerschnittes oder dgl., ermöglicht, und zwar ohne die Verwendung zusätzlicher Hilfsmittel, wie z.B. Unterlegsscheiben etc.

Gelöst wird diese Aufgabe erfindungsgemäß durch die Gestaltung des Implantats mit den kennzeichnenden Merkmalen des Anspruches 1 und in weiterer Ausgestaltung durch die Merkmale der Unteransprüche 2 bis 7.
Anhand der Zeichnungsfiguren 3 bis 11 wird das erfindungsgemäße Implantat in der folgenden Beschreibung näher erläutert.
Es zeigt:
- Fig. 1: ein bekanntes bandartiges Implantat in Vorderansicht;
- Fig. 2: das bandartige Implantat der Fig. 1 in Seitenansicht;
- Fig. 3: das bandartige Implantat der Fig. 1 und 2 in Draufsicht;
- Fig. 4: ein erfindungsgemäßes, an zwei Halswirbeln montiertes Implantat in Vorderansicht;
- Fig. 5: eine Seitenansicht aus Fig. 4;
- Fig. 6: Einzelteile des Implantats der Fig. 4 und 5 in axonometrischer Explosionsdarstellung;
- Fig. 7: eine Montageplatte des Implantats der Fig. 4 bis 6 in Draufsicht;
- Fig. 8: das Implantat der Fig. 4 in einer veränderten Montageposition in Vorderansicht;
- Fig. 9: die an einem Halswirbel montierte Montageplatte der Fig. 7 in Draufsicht;
- Fig. 10: eine andere Ausführungsform der an einem Halswirbel montierten Montageplatte in Vorderansicht und
- Fig. 11: ein an drei Halswirbeln montiertes Implantat mit mehreren gelenkigen Lagern in Vorderansicht.

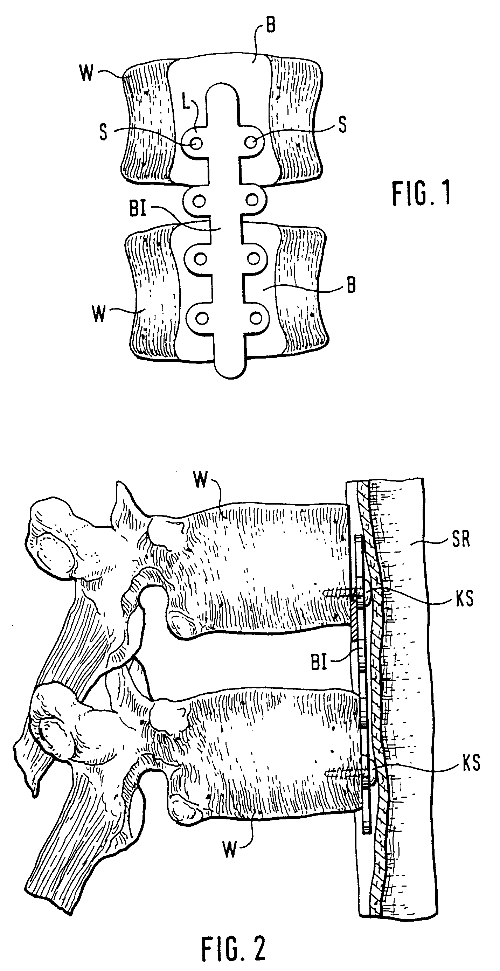

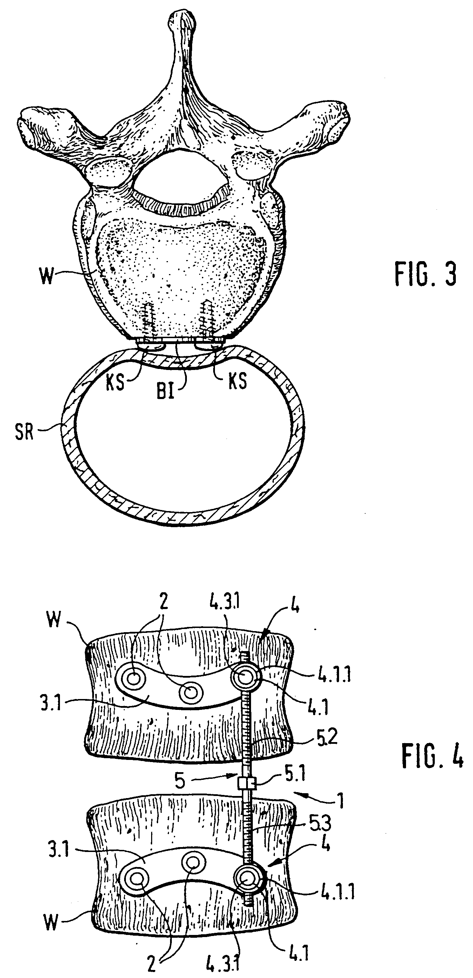

Die Fig. 1, 2, 3 zeigen ein bekanntes bandartiges Implantat BI, das im wesentlichen aus einem Metallband M besteht, an welchem seitliche Laschen L angeordnet sind, wobei das Metallband M wie auch die Laschen L Schraubenbohrungen S aufweisen, welche zur Aufnahme von Knochenschrauben KS dienen und entsprechend ausgebildet sind. Die Schraubenbohrungen S bzw. die diese aufweisenden Laschen L sind in festen Abständen angeordnet. Es gibt diese Metallbänder M in verschiedenen Längen, damit sie entsprechend zu den jeweiligen körperlichen Verhältnissen eines Patienten passend ausgewählt werden können. Ein solches bandartiges Implantat BI benötigt ein Bett B auf dem Wirbelknochen W, damit es montiert werden kann. Die Montage erfolgt durch Anschrauben des bandartigen Implantates an den Wirbelknochen W. Das Bett B wird hergestellt, indem der Wirbelknochen W beispielsweise abgefräst wird. Ebenso muß auch auf dem benachbarten, zu fixierenden Wirbelknochen W ein Bett B geformt werden, um hernach diesen Wirbelknochen W mit dem bandartigen Implantat BI in der medizinisch erforderlichen Wirbelposition zu verschrauben. In den Fig. 2 und 3 ist die durch das bandartige Implantat BI verursachte Verengung der Speiseröhre SR angedeutet. Zudem ist in Fig. 2 die Fixation mit einem bandartigen Implantat BI mittels eines Unterlegplättchens dargestellt.

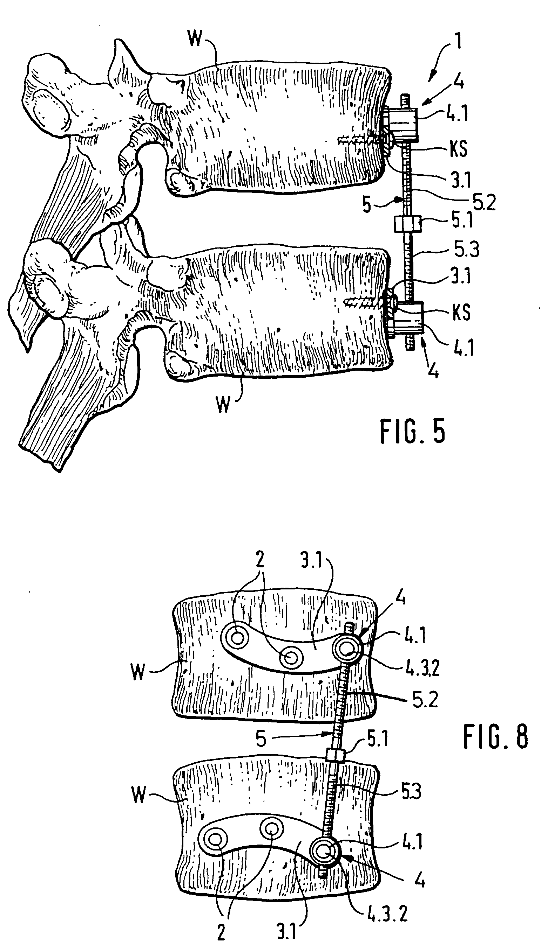

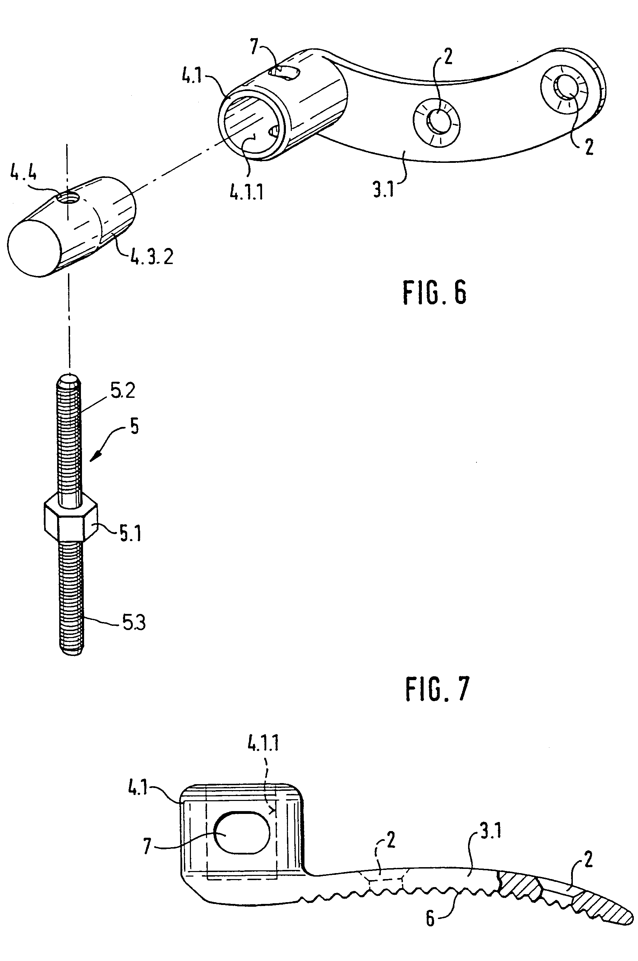

Das in den Fig. 4 bis 11 dargestellte erfindungsgemäße Implantat 1 besteht aus wenigstens zwei Montageplatten 3.1 bzw. 3.2 oder 3.3, welche mittels eines jeweils einstückig angeformten gelenkigen Lagers 4 bzw. 4.2, mit einer in diese Lager 4 bzw. 4.2 eingeschraubten Spindel 5 verstellbar verbunden sind.
Die Spindel 5 weist ein links- und ein rechtsgängiges seöbsthemmendes Gewinde 5.2 und 5.3 auf, zwischen denen ein kantiges Schaftstück 5.1 zur Spindelbetätigung, z.B. mit einem Sechskantschlüssel oder dgl., angeordnet ist. Dabei kann es vorteilhaft sein, insbesondere wenn die Spindel 5 mit einem ihrer Gewinde 5.2, 5.3 permanent in je einem Lagerbolzen 4.3 eingeschraubt ist und am entsprechenden Gewindeende als Ausdrehsicherung eine Verquetschung aufweist.

Die Montageplatte 3.1 ist als nierenförmige, gewölbte Platte ausgebildet, welche an einem ihrer Enden das gelenkige Lager 4 aufweist. Sie weist benachbart zum gelenkigen Lager 4 wenigstens zwei Schraubenlöcher 2 auf, welche angesenkt sind. Auf der dem gelenkigen Lager 4 abgewandten Seite weist die nierenförmige, gewölbte Platte eine Riffelung 6 auf, mit der einerseits die bei Bewegungen des Patienten aus der gegenseitigen Verdrehung der Wirbel auftretenden und andererseits die durch die mittels der Spindel 5 erzeugten Verspannung der Wirbelknochen W entstehenden Kräfte besser und nicht allein von den Knochenschrauben in den Schraubenlöchern 2 aufgenommen werden können.

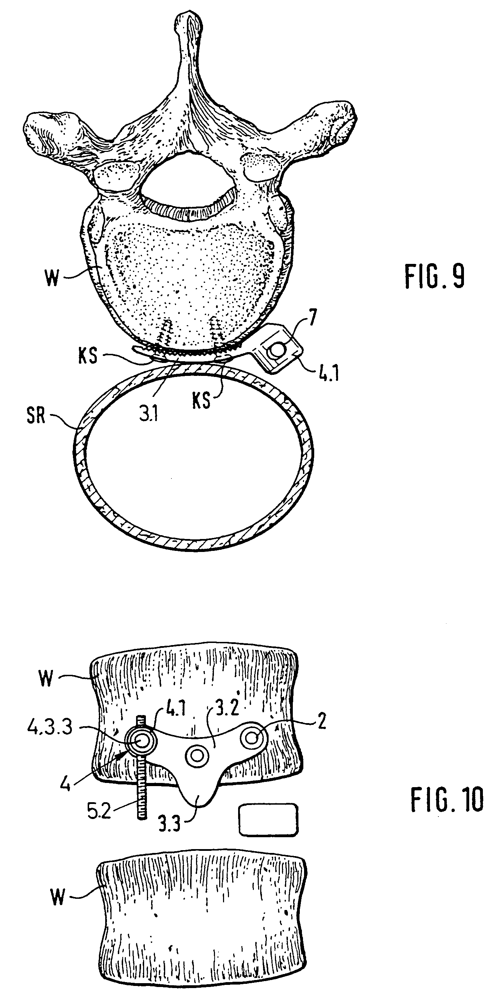

Die Montageplatte 3.2 gemäß Fig. 10 ist im wesentlichen genau wie die Montageplatte 3.1 ausgebildet, doch weist sie zusätzlich einen Haltelappen 3.3 auf, welcher quer zu ihrer Längserstreckung von ihr absteht und dazu dient, ein gegebenenfalls zwischen den Wirbeln eingesetzes Wirbeldistanzstück oder ein sonstiges, chirurgisch erforderliches zusätzliches Implantat gegen Herausrutschen aus dem Raum zwischen den Wirbeln zu sichern.

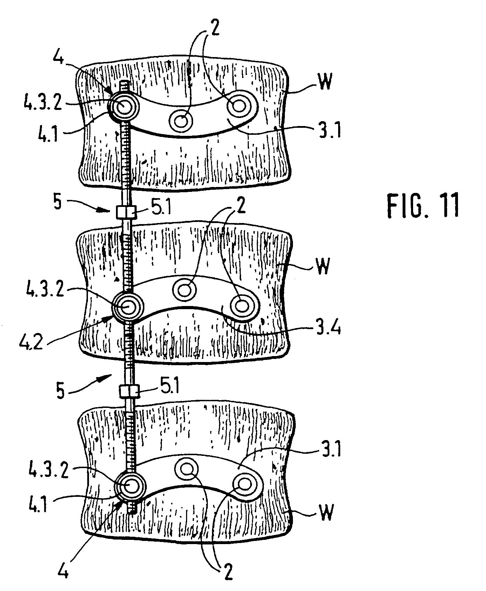

In einer weiteren Ausführungsform (Fig. 11) kann die im übrigen wie die Montageplatten 3.1 oder 3.2 ausgebildete Montageplatte 3.4 zwei gelenkige Lager 4 in geeigneter Anordnung nebeneinander aufweisen. Eine solche mit einem doppelgelenkigen Lager 4.2 versehene Montageplattte 3.4 kann insbesondere zum Verbinden von drei Wirbelknochen W vorgesehen werden, indem beidseits dieser doppelgelenkigen Montageplatte je eine Montageplatte 3.1 oder 3.2 an den entsprechenden, benachbarten Wirbelknochen W befestigt ist und die beiden Montageplatten 3.1 oder 3.2 mit der doppelgelenkigen Montageplatte 3.4 jeweils durch eine Spindel 5 miteinander verbunden sind.

Das gelenkige Lager 4 besteht in einer ersten Ausführungsform aus wenigstens einer einstückig an der Montageplatte 3.1 bzw. 3.2 angeformten Lagerbuchse 4.1, welche eine im wesentlichen quer zur Längserstreckung der Montageplatte 3 verlaufende Spindelbohrung 7 für den Durchtritt der Spindel 5 und eine zylindrische Innenwand 4.1.1 aufweist. In diese Lagerbuchse 4.1 ist ein zylindrischer Lagerbolzen 4.3.3 einsetzbar, der eine Bohrung 4.4 mit einem Innengewinde zum Einschrauben der Spindel 5 aufweist.

In einer zweiten Ausführungsform des gelenkigen Lagers 4 ist in die Lagerbuchse 4.1 ein doppelkegelstumpfförmiger Lagerbolzen 4.3.3 einsetzbar. Dieser weist ebenfalls eine Bohrung 4.4 mit einem Innengewinde zum Einschrauben der Spindel 5 auf.

In einer dritten Ausführungsform des gelenkigen Lagers 4 ist in die Lagerbuchse 4.1 ein tonnenförmiger Lagerbolzen 4.3.3 einsetzbar, der wiederum die Bohrung 4.4 mit einem Innengewinde zum Einschrauben der Spindel 5 aufweist.

Bei einer Kompression oder einer Distraktion (Fig. 4,5, 6) zweier benachbarter Wirbelknochen W wird eine Montageplatte 3.1 oder eine Montageplatte 3.2 mit einem Wirbelknochen W mittels Knochenschrauben K verschraubt. Ebenso wird eine Montageplatte 3.1 oder eine Montageplatte 3.2 mit einem zweiten Wirbelknochen W mittels Knochenschrauben K verschraubt. Die beiden Montageplatten 3.1 bzw. 3.2 werden anschließend mittels der Spindel 5 verbunden. Durch Verdrehen der Spindel 5 werden die beiden Wirbelknochen W zueinander justiert und fixiert. Dabei können die Montageplatten 3.1, 3.2 mit den gelenkigen Lagern 4 etwas seitlich neben der Speiseröhre SR an den Wirbelknochen W montiert werden, so daß sich keine oder nur eine vernachlässigbare Beeinträchtigung des Patienten beim Schlucken ergibt. Die Montageplatten 3.2 werden vorzugsweise dann verwendet, wenn zwischen den Wirbelknochen W noch andere operative Hilfsmittel, z.B. zur Stützung der Wirbelknochen W, verwendet werden müssen, die jedoch den Raum zwischen den beiden Wirbelknochen W nicht verlassen dürfen und von der Montageplatte 3.2 gehalten werden können (Fig. 10).

Bei einer Kompression oder einer Distraktion von drei benachbarten Wirbelknochen W (Fig. 11) werden an den beiden randseitig liegenden Wirbelknochen W Montageplatten 3.1 oder 3.2 montiert, wogegen auf dem mittleren Wirbelknochen W eine Montageplatte 3.4 mit einem doppelgelenkigen Lager 4.2 angeschraubt wird. Mit je einer Spindel 5 wird auf jeder Seite des mittleren Wirbelknochens W die entsprechende Montageplatte 3.1 bzw. 3.2 des benachbarten Wirbelknochens W justiert und fixiert.

Es ist aber auch möglich (Fig. 8), die beiden Montageplatten 3 zweier benachbarter Wirbelknochen W unter beliebigem, sich aus einer gegebenen Verletzungssituation der Wirbel ergebendem Winkel zu montieren und mit einer beispielsweise schräg liegenden Spindel 5 zu verbinden und zu justieren. Dadurch sind bei sehr vielen verschiedenen Verletzungen der Wirbelknochen W immer noch Fixationen derselben möglich, wodurch die Heilungschancen und die Heilungsqualitäten wesentlich vergrößert werden. Gegenüber dem aus der US-PS 4771 737 bekannten Implantat hat das erfindungsgemäße Implantat den erheblichen Vorteil, daß die Montageplatte mit minimaler Raumbeanspruchung mittels zweier Knochenschrauben fest am jeweiligen Wirbel, u.z. vorderseitig fixiert werden kann, u.z. so, daß eine Änderung der Lage am Wirbel ausgeschlossen ist. Während bei der bekannten Ausführungsform die Befestigung der Montageblöcke mittels einer Gewindemutter erfolgt, die zugleich die beiden Blockhkälften zusammenhält, zwischen welche ein zylindrischer Lagerbolzen oder eine mit einer Gewindebohrung versehene Kugel als Teil eines Gelenklagers angeordnet sind, ist beim erfindungsgemäßen Implantat die Lagerbuchse 4.1 bzw. 4.2 einstückig und somit starr mit der Montageplatte 3.1, 3.2 bzw. 3.4 verbunden. Dadurch läßt sich die Montageplatte mit der Lagerbuchse auch bei kleinster Bauweise mit der jeweils größtmöglichen Festigkeit herstellen, die für eine exakte Justierung der Wirbel von großer Bedeutung ist. DAbei ist es auch wichtig, daß die Montageplatte, die ja nur eine relativ kleine Auflagefläche aufweist, auf diese Auflagefläche mit einer Riefelung versehen ist, damit sie am Wirbelknochen einen unveränderlichen, festen Sitz erhält.
Der größte Vorteil des erfindungsgemäßen Implantats gegenüber dem bekannten Implantat besteht aber darin, daß die Wirbel, an denen die Montageplatten 3.1, 3.2 bzw. 3.4 anzuschrauben sind, bevor nicht nicht bearbeitet werden müssen und daß die relativ dünne Platte störungsfrei auf der der Speiseröhre SR zugekehrten Seite der Wirbel angebracht werden kann u.z. so, daß sich die Lagerbuchse seitlich neben der Speiseröhre SR befindet.

Gegenüber der mit dem bekannten bandartigen Implantat BI erreichbaren Fixation der Wirbelknochen W ist mit dem erfindungsgemäßen Implantat 1 eine Kompression oder eine Distraktion erreichbar, wodurch die Anwendungsmöglichkeiten des Implantats wesentlich erweitert sind. Darüberhinaus ist das Implantat mit der Montageplatte 3.2 aber auch als eine Haltehilfe anstatt eines normalerweise zusätzlich erforderlichen chirurgischen Instrumentes einsetzbar, da es die Wirbel während anderer operativer Maßnahmen schon in einer für die Operation günstigen Lage halten kann.

Insbesondere können die beiden Wirbelknochen W durch die Spindel 5 sehr genau zueinander justiert werden, was beim bandartigen Implantat nach dem Stand der Technik nicht möglich ist. Besonders vorteilhaft ist aber, daß die Montage des erfindungsgemäßen Implantates 1 ohne jegliche Vorarbeiten am Wirbelknochen W vorgenommen werden kann, wodurch einerseits die Operationszeit und damit auch die Narkosezeit des Patienten und andererseits auch die Größe des Operationsfeldes, d.h. also die Wunde, kleiner gehalten werden können. Beides nicht unerhebliche Fortschritte in der chirurgischen Behandlung bei ohnehin kritischen Operatonen.

Ein wesentlicher Vorteil des erfindungsgemäßen Implantats liegt außerdem in der seitlichen Anordnung des gelenkigen Lagers 4, welche es erlaubt, dieses vorspringende Bauteil in eine natürliche Wirbelkörpernische zu plazieren und damit insbesondere die behindernde Deformation der Speiseröhre zu vermeiden.

## Patentansprüche

1. Mehrteiliges Implantat zur Fixierung der Wirbel (W), insbesondere der Halswirbel, auf deren Vorderseite, d.h. speiseröhrenseitig, bestehend aus wenigstens zwei Montageteilen (3.1), die zur Verschraubung mit einem Wirbel (W) Schraubenlöcher (2) aufweisen und die jeweils durch gelenkige Lager (4) und eine ein links- und ein rechtsgängiges Gewinde (5.2, 5.3) aufweisende Spindel (5) verstellbar miteinander verbunden sind, wobei die Lager (4) jeweils einen Lagerbolzen (4,3) mit einer quer zu seiner Schwenkachse verlaufenden Bohrung (4.4) mit einem Innengewinde zur Aufname eines Gewindes (5.2, 5.3) der Spindel (5) aufweisen und die Spindel (5) zu ihrer Betätigung mit einem kantigen Schaftstück (5.1) versehen ist,
**dadurch gekennzeichnet,**.
daß die Montageteile jeweils als im wesentlichen nierenförmige, gewölbte Montageplatten (3.1, 3.2) ausgebildet sind, die jeweils mit wenigstens zwei zum gelenkigen Lager (4) benachbarten Schraubenlöchern (2) und auf der dem Wirbelknochen (W) zugewandten Seite mit einer Riffelung (6) versehen sind und daß die gelenkigen Lager (4) jeweils aus einer am einen Ende einer Montageplatte (3.1, 3.2) einstückig angeformten Lagerbuchse (4.1) mit einer sich im wesentlichen quer zur Längserstreckung der Montageplatte (3) erstreckenden Spindelbohrung (7) für den Durchtritt der Spindel (5) und einem Lagerbolzen bestehen, wobei jeweils der die Bohrung (4.4) mit einem Innengewinde zur Aufnahme eines Gewindes (5.2, 5.3) der Spindel (5) aufweisende Lagerbolzen (4.3.1, 4.3.2, 4.3.3) drehbar in der Lagerbuchse (4.1) gelagert ist.

2. Implantat nach Anspruch 1, dadurch gekennzeichnet, daß die Montageplatte (3.2) einen Haltelappen (3.3) aufweist, der quer zu ihrer Längserstreckung von ihr absteht.

3. Implantat nach Anspruch 1 oder 2, dadurch gekennzeichnet, daß die Lagerbuchse (4.1) eine zylindrische Innenwand (4.1.1) aufweist und der Lagerbolzen (4.3.1) zylindrisch ist.

4. Implantat nach einem der Ansprüche 1 bis 3, dadurch gekennzeichnet, daß die Lagerbuchse (4.1) eine zylindrische Innenwand (4.1.1) aufweist, in die ein doppelkegelstumpfförmiger Lagerbolzen (4.3.2) mit einer Bohrung (4.4) mit einem Innengewinde zum Einschrauben eines Gewindes (5.2, 5.3) der Spindel (5) einsetzbar ist.

5. Implantat nach Anspruch 2 oder 3, dadurch gekennzeichnet, daß die Lagerbuchse (4.1) eine zylindrische Innenwand (4.1.1) aufweist, in die ein tonnenförmiger Lagerbolzen (4.3.3.) mit einer Bohrung (4.4) mit einem Innengewinde zum Einschrauben eines Gewindes (5.2, 5.3) der Spindel (5) einsetzbar ist.

6. Implantat nach einem der Ansprüche 1 bis 5, dadurch gekennzeichnet, daß die Gewinde (5.2, 5.3) der Spindel (5) selbsthemmend sind.

7. Implantat nach einem der Ansprüche 1 bis 6, dadurch gekennzeichnet, daß die Spindel (5) mit ihren freien Enden permanent in den Lagerbolzen (4.3) eingeschraubt ist und am Gewindeende als Ausdrehsicherung eine Verquetschung aufweist.

## Claims

1. Multi-component implant for fixing the vertebrae (W), in particular the cervical vertebrae, at their front, i.e. oesophagus side consisting of at least two assembly members (3.1) which have screw holes (2) for screwing to a vertebra (W) and are adjustably connected to one another in each case by articulated bearings (4) and a spindle (5) having a left-handed and a right-handed thread (5.2, 5.3), wherein the bearings (4) have a respective bearing journal (4.3) with a bore (4.4) extending transversely to its pivot axis with an internal thread for receiving a thread (5.2, 5.3) of the spindle (5) and the spindle (5) is provided with an angular shank member (5.1) for the actuation thereof, characterised in that the assembly members are designed in each case as substantially kidney-shaped curved assembly plates (3.1, 3.2) which are each provided with at least two screw holes (2) adjacent to the articulated bearing (4) and are provided on the side facing the vertebral bone (W) with corrugations (6) and that the articulated bearings (4) consist in each case of a bearing bush (4.1) shaped integrally on one end of an assembly plate (3.1, 3.2) with a spindle bore (7), extending substantially transversely to the longitudinal direction of the assembly plate (3), for the passage of the spindle (5), and of a bearing journal, wherein the respective bearing journal (4.3.1, 4.3.2, 4.3.3) having the bore (4.4) with an internal thread for receiving a thread (5.2, 5.3) of the spindle (5) is rotatably mounted in the bearing bush (4.1).

2. Implant according to claim 1, characterised in that the assembly plate (3.2) has a holding tab (3.3) which projects from it transversely to its longitudinal direction.

3. Implant according to claim 1 or 2, characterised in that the bearing bush (4.1) has a cylindrical internal wall (4.1.1) and the bearing journal (4.3.1) is cylindrical.

4. Implant according to one of claims 1 to 3, characterised in that the bearing bush (4.1) has a cylindrical internal wall (4.1.1) into which a double truncated cone-shaped bearing journal (4.3.2) with a bore (4.4) having an internal thread for screwing in of a thread (5.2, 5.3) of the spindle (5) can be inserted.

5. Implant according to claim 2 or 3, characterised in that the bearing bush (4.1) has a cylindrical internal wall (4.1.1) into which a barrel-shaped bearing journal (4.3.3) with a bore (4.4) having an internal thread for screwing in of a thread (5.2, 5.3) of the spindle (5) can be inserted.

6. Implant according to one of claims 1 to 5, characterised in that the threads (5.2, 5.3) of the spindle (5) are self-locking.

7. Implant according to one of claims 1 to 6, characterised in that the spindle (5) is screwed permanently into the bearing journal (4.3) by its free ends and has a squeezing means at the thread end as a release-preventing means.

## Revendications

1. Implant en plusieurs parties destiné à la fixation des vertèbres (W), en particulier des vertèbres cervicales, sur leur côté avant, c'est-à-dire du côté de l'oesophage, et constitué d'au moins deux éléments de montage (3.1) qui en vue de leur assujettissement à une vertèbre (W) par vissage présentent des trous à vis (2) et qui, au moyen d'articulations (4) et d'une broche (5) présentant un filetage à gauche et un filetage à droite (5.2, 5.3), sont reliés entre eux de manière réglable, les articulations (4) présentant chacune une cheville (4.3) avec un alésage (4.4) qui, s'étendant transversalement à l'axe de pivotement de cette dernière, présente un taraudage pour recevoir un filetage (5.2, 5.3) de la broche (5), et la broche (5) étant munie, en vue de son actionnement, d'une pièce de fût polygonale (5.1), caractérisé en ce que les éléments de montage sont chacun réalisés sous la forme d'une plaquette de montage (3.1, 3.2) courbée sensiblement en forme de rein qui présentent chacune au moins deux trous à vis (2) voisins de l'articulation (4) et, du côté de l'os vertébral (W), des striures (6) et en ce que les articulations (4) sont chacune constituées par une douille de montage (4.1) qui, rapportée à une extrémité d'une plaquette de montage (3.1, 3.2) et réalisée en une pièce avec cette dernière, présente un trou à broche (7), s'étendant sensiblement transversalement à l'étendue longitudinale de la plaquette de montage (3), pour le passage de la broche (5), et par une cheville, laquelle cheville (4.3.1, 4.3.2, 4.3.3) présentant l'alésage (4.4) muni d'un taraudage pour recevoir un filetage (5.2, 5.3) de la broche (5) est montée de façon à pouvoir tourner dans la douille de montage (4.1).

2. Implant selon la revendication 1, caractérisé en ce que la plaquette de montage (3.2) présente une patte de retenue (3.3) qui fait saillie sur la plaquette de montage transversalement à l'étendue longitudinale de celle-ci.

3. Implant selon la revendication 1 ou 2, caractérisé en ce que la douille de montage (4.1) présente une paroi intérieure cylindrique (4.1.1) et en ce que la cheville (4.3.1) est cylindrique.

4. Implant selon l'une quelconque des revendications précédentes, caractérisé en ce que la douille de montage (4.1) présente une paroi intérieure cylindrique (4.1.1) dans laquelle peut être mise en place une cheville (4.3.2) en forme de double cône tronqué présentant un alésage (4.4) avec un taraudage permettant d'y visser un filetage (5.2, 5.3) de la broche (5).

5. Implant selon la revendication 2 ou 3, caractérisé en ce que la douille de montage (4.1) présente une paroi intérieure cylindrique dans laquelle peut être mise en place une cheville (4.3.3) en forme de tonneau présentant un alésage (4.4) avec un taraudage permettant d'y visser un filetage (5.2, 5.3) de la broche (5).

6. Implant selon l'une quelconque des revendications précédentes, caractérisé en ce que les filetages (5.2, 5.3) de la broche (5) sont autobloquants.

7. Implant selon l'une quelconque des revendications précédentes, caractérisé en ce que la broche (5) est vissée avec ses extrémités libres de façon permanente dans les chevilles (4.3) et présente à l'extrémité filetée un écrasement pour empêcher la broche de sortir des chevilles en tournant.
